# FASCICULE DE BREVET EUROPEEN

(11) **EP 4 007 580 B1**
(45) Date de publication et mention de la délivrance du brevet: **07.06.2023**
(21) Numéro de dépôt: 20746623.6
(22) Date de dépôt: 24.07.2020
(51) Int. Cl.: A61K 31/575, A61P 25/00, A61P 43/00

(54) **COMPOSITION COMPRENANT DES DÉRIVÉS DE STÉROLS POUR SON UTILISATION DANS LE TRAITEMENT D'UNE PATHOLOGIE NEURONALE LIÉE À UNE HYPOXIE, À UNE HYPOGLYCÉMIE ET/OU UNE HYPERGLYCÉMIE**
ZUSAMMENSETZUNG MIT STEROLDERIVATEN ZUR VERWENDUNG BEI DER BEHANDLUNG EINER NEURONALEN ERKRANKUNG IM ZUSAMMENHANG MIT HYPOXIE, HYPOGLYKÄMIE UND/ODER HYPERGLYKÄMIE
COMPOSITION COMPRISING STEROL DERIVATIVES FOR USE IN THE TREATMENT OF A NEURONAL PATHOLOGY RELATED TO HYPOXIA, HYPOGLYCEMIA AND/OR HYPERGLYCEMIA

(30) Priorité: 01.08.2019 BE 201905501
(43) Date de publication de la demande: 08.06.2022
(73) Titulaire: Dendrogenix, 4000 Liège (BE)
(72) Inventeur: SILVENTE, Stéphane, 4000 LIEGE (BE); MARLIER, Quentin, 4000 LIEGE (BE); RIVES, Arnaud, 4000 LIEGE (BE); CARON, Nicolas, 4000 LIEGE (BE)
(74) Mandataire: Loyer & Abello
(86) Numéro de dépôt international: PCT/EP2020/071044
(87) Numéro de publication internationale: WO 2021/018798

(56) Documents cités:
- WO-A2-03/089449
- WO-A2-2016/016518
- FR-A1- 2 981 351
- DE MEDINA PHILIPPE ET AL: "Synthesis of new alkylaminooxysterols with potent cell differentiating activities: identification of leads for the treatment of cancer and neurodegenerative diseases", JOURNAL OF MEDICINAL CHEMISTRY, AMERICAN CHEMICAL SOCIETY, US, vol. 52, no. 23, 10 décembre 2009 (2009-12-10), pages 7765-7777, XP009131948, ISSN: 0022-2623, DOI: 10.1021/JM901063E [extrait le 2009-09-22] cité dans la demande
- BRIGITTE MALGRANGE ET AL: "Targeting Cholesterol Homeostasis to Fight Hearing Loss: A New Perspective", FRONTIERS IN AGING NEUROSCIENCE, vol. 7, 29 janvier 2015 (2015-01-29), XP055687450, DOI: 10.3389/fnagi.2015.00003
- ZHAO H-B ET AL: "Ganoderma total sterol (GS) and GS"1 protect rat cerebral cortical neurons from hypoxia/reoxygenation injury", LIFE SCIENCES, PERGAMON PRESS, OXFORD, GB, vol. 76, no. 9, 14 janvier 2005 (2005-01-14), pages 1027-1037, XP004686351, ISSN: 0024-3205, DOI: 10.1016/J.LFS.2004.08.013

## Description

### Domaine technique

L'invention se rapporte au domaine des pathologies neuronales liées à la déprivation de glucose et/ou d'oxygène. Plus précisément, l'invention concerne une composition comprenant des composés dérivés de stérols de formule (I) ou un sel pharmaceutiquement acceptable d'un tel composé, pour son utilisation dans le traitement et/ou la prévention d'une ischémie cérébrale due à un accident vasculaire cérébral, un traumatisme crânien, une lésion cérébrale, une hypoglycémie liée à un diabète, une hyperglycémie ou un problème respiratoire dû notamment à une infection bactérienne ou virale.

### Arrière-plan technologique

Une ischémie est une diminution de l'apport sanguin à un organe, l'ischémie cérébrale est donc la diminution de l'apport sanguin à au moins une partie du cerveau. L'ischémie provoque par conséquent l'interruption à la fois de l'apport d'oxygène, de l'apport en nutriments, comme le glucose, et de l'élimination des molécules toxiques résultant du métabolisme anaérobique.

L'ischémie cérébrale est impliquée notamment dans les accidents vasculaires cérébraux (AVC) communément appelés "attaques cérébrales", et les traumatismes crâniens.

Les AVC représentent la troisième cause de mortalité derrière les maladies cardiovasculaires et le cancer. Mais ce sont les premières causes d'invalidité lourde et les deuxièmes causes de démence dans le monde occidental. A l'origine des AVC, on trouve une perturbation de l'irrigation sanguine du cerveau. Près de 85 % des AVC sont de nature ischémique c'est à dire dus à l'obstruction d'un vaisseau sanguin par un caillot, réduisant l'irrigation sanguine dans une zone cérébrale. Le caillot peut se former localement dans une petite artère ou venir du coeur ou d'une lésion de la paroi d'une des grosses artères cervicales. Cette obstruction empêche donc un apport suffisant en oxygène et en métabolites, comme le glucose, pour répondre à la demande métabolique et énergétique du système nerveux central, et provoque une ischémie cérébrale. Les conséquences sont d'autant plus importantes que le cerveau n'est pas en mesure de passer d'un métabolisme aérobie à un métabolisme anaérobie pour produire l'énergie dont il a besoin. Ainsi certaines cellules du cerveau peuvent mourir.

Les conséquences des AVC dépendent de nombreux facteurs : la vitesse de rétablissement de la circulation sanguine, la durée de la privation en oxygène et/ou en métabolites, comme le glucose, et/ou la localisation cérébrale de l'accident. Les manifestations cliniques se traduisent, selon le territoire atteint, par une paralysie plus ou moins étendue, une perte de la parole ou du langage, ou encore un coma, avec des risques séquellaires invalidants.

En outre, une insuffisance respiratoire due notamment à une infection bactérienne ou virale peut également entrainer une ischémie cérébrale. Par exemple, un AVC ischémique peut se déclencher lorsqu'une inflammation de vaisseaux sanguins (vascularite) ou une infection (à virus herpès simplex, par exemple) rétrécit les vaisseaux sanguins qui approvisionnent le cerveau. De plus en plus d'éléments tendent à démontrer également qu'il existe par exemple un impact négatif du Covid-19 sur le système nerveux. Différentes études ont en effet montré qu'il existait une corrélation entre infection au Covid-19 et perte de gout et d'odorat, confusion, maux de tête, étourdissements voire même AVC, encéphalopathie ou encore myélite (Ling Mao et al., Neurogical Manifestations of Hospitalized Patients with COVID-19 in Wuhan, china: a rétrospective case series study et Neo Poyiadji et al COVID-19-associated Acute Hemorrhagic Necrotizing Encephalopathy: CT and MRI, <doi : https://doi.org/10.1101/2020.02.22.20026500>). Le virus serait en effet capable d'entrer dans le système nerveux via les fosses nasales et le bulbe olfactif. Des récepteurs au Covid-19, que l'on retrouve massivement dans les poumons, se retrouvent d'ailleurs également au niveau de la barrière hémato-encéphalique et des terminaisons nerveuses. Une fois dans le système nerveux, il est possible que le virus détruise les neurones responsables du bon fonctionnement de la respiration. La destruction des neurones du système nerveux autonome présents dans le bulbe rachidien, entraine et/ou aggrave les détresses respiratoires des patients. A contrario, il est par ailleurs possible qu'un niveau d'oxygène bas dans le sang dû aux symptômes respiratoires aigus des patients atteints du Covid-19 soit lui-même responsable des troubles neurologiques. Les neurones étant très sensibles au manque d'oxygène, une diminution prolongée et prononcée du taux d'oxygène sanguin altérerait les neurones qui finiraient par mourir.

Une autre cause d'ischémie cérébrale est également le traumatisme crânien qui peut être bénin ou grave, avec tous les états intermédiaires possibles. Sa gravité dépend de l'existence de lésions intra-cérébrales ou de l'existence d'un hématome extra-cérébral, saignement situé entre le crâne et le cerveau. Un traumatisme crânien peut s'accompagner de contusions, de lésions des neurones, d'un oedème, d'hémorragies intra-cérébrales et/ou d'une ischémie.

L'ischémie cérébrale est la menace majeure qui pèse sur le devenir fonctionnel et anatomique du cerveau traumatisé. Il s'agit là d'une ischémie globale, diffuse, ou multicentrique, qui touche le cortex cérébral comme l'aurait fait une anoxie ou un arrêt cardiaque. L'ischémie touche l'ensemble de la substance grise, partie des tissus du système nerveux central ayant la demande métabolique la plus forte en oxygène et en glucose. Toutes les fonctions cérébrales sont ainsi menacées. La perte cellulaire peut alors être massive et marquée par une atrophie cérébrale visible.

L'ischémie peut prendre une autre forme plus locale. La microcirculation des tissus situés autour d'un foyer de contusion ou d'hémorragie est menacée par une vasoconstriction, l'effet d'une compression tissulaire, des micro-thromboses capillaires, ou encore des désordres du métabolisme cellulaire. La production d'énergie de la cellule est compromise car son métabolisme oxydatif est en panne. Des produits toxiques comme les radicaux libres sont libérés. Le moindre événement respiratoire ou circulatoire peut alors précipiter les cellules en dessous du seuil de viabilité.

Les conséquences des traumatismes crâniens peuvent être des atteintes physiques de type paraplégie, hémiplégie, troubles de la vue... Elles peuvent être aussi des atteintes neuropsychologiques affectant la mémoire, l'attention, la capacité à communiquer. Enfin elles modifient le comportement et la personnalité du blessé et son quotidien, ainsi que celui de ses proches.

Certains cas de diabète présentent une hypoglycémie couplée à une hypoxie dont les conséquences sont délétères pour les patients touchés.

L'hypoglycémie est la complication clinique la plus répandue dans la gestion quotidienne des diabétiques traités à l'insuline, et continue d'être le facteur limitant dans la gestion glycémique du diabète. L'hypoglycémie sévère touche 40% des diabétiques traités à l'insuline et peut entrainer des lésions cérébrales, en particulier au niveau des neurones vulnérables du cortex et de l'hippocampe. Par exemple, les déficits d'apprentissage et de mémoire sont une conséquence directe de cette lésion neuronale de l'hippocampe provoquée par une hypoglycémie sévère.

Il est connu que l'hyperglycémie est un des symptômes révélateurs du diabète. En effet, le diabète entraine systématiquement une hyperglycémie. En outre, une hyperglycémie non liée au diabète existe également et les fait en causes sont variés, par exemple, l'ingestion d'aliments ou de boissons contenant des sucres ou les effets secondaires d'un médicament. L'hyperglycémie liée à un diabète ou non induit des effets délétères au niveau du système nerveux.

De plus, l'association d'un diabète et d'une hyperglycémie aggrave les dommages neuronaux consécutifs à d'autres formes d'atteinte du système nerveux central, comme les accidents vasculaires cérébraux. Plusieurs études démontrant la relation entre hyperglycémie, diabète et dommages neuronaux ont été publiées, notamment Yazi Li et al. (« Autophagy impairment mediated by S-nitrosation of ATG48 leads to neurotoxicity in response to hyperglycemia », <doi :https://doi.org/10.1080/15548627.2017.1320467>), Ruchi Sharma et al. (« Hyperglycemia Induces Oxidative Stress and Impairs Axonal Transport Rates in Mice », Published October 18, 2010*) et* Wenjuan Zhou et al. (« TIGAR Attenuates High Glucose-Induced Neuronal Apoptosis via an Autophagy Pathway », <doi : https://doi.org/10.3389/fnmol.2019.00193>).

Actuellement, les techniques développées pour traiter un AVC sont d'intervenir très rapidement, dans les 4 heures à 5 heures suivant l'AVC et d'éliminer le caillot chimiquement via un activateur tissulaire du plasminogène recombinant, rtPA ou mécaniquement, par thrombectomie pour permettre la reperfusion. Cependant, ces deux façons d'intervenir face à un AVC ne présentent pas une efficacité optimale et sont soumis à des critères d'inclusion des patients assez restrictifs.

On connaît du document WO2016016518A2 une composition comprenant dans un véhicule pharmaceutiquement acceptable au moins un composé de formule (I) pour prévenir une perte d'audition chez un sujet ou pour obtenir une restauration au moins partielle de l'audition d'un sujet traité ayant, avant traitement, une fonction auditive réduite.

On connaît également du document de Zhao H-B et al. (« Ganoderma total sterol (GS) and GS1 protect rat cérébral cortical neurons from hypoxia/reoxygenation injury », <doi :https://doi.org/10.1016/j.Ifs.2004.08.013>), l'effet du *Ganoderma total stérol* (GS) et de ses principaux composants (GS1) sur les cultures de neurones corticaux de rats exposés à l'hypoxie / réoxygénation.

On connaît encore du *document* Brigitte Malgrange et al. (« Targeting Cholestérol Homeostasis to Fight Hearing Loss: A New Perspective », <doi: 10.3389/fnagi.2015.00003>), un moyen de lutte contre la perte auditive en ciblant l'homéostasie du cholestérol. En outre, il est indiqué que le rôle du cholestérol et de ses métabolites n'est pas clair.

Les trois documents précités ne divulguent pas ni ne suggèrent une composition comprenant au moins un composé de formule (I) pour son utilisation dans le traitement d'une pathologie neuronale d'un sujet, ladite pathologie neuronale étant liée à une hypoxie et/ou à une hypoglycémie affectant des cellules du système nerveux central.

Il existe donc un besoin de développer des composés permettant de traiter des patients ayant subis une déprivation en oxygène et/ou en glucose, provoqué par exemple par une ischémie cérébrale, une hypoglycémie ou une hypoxie, afin d'améliorer leur récupération.

### Résumé

Une idée à la base de l'invention est de fournir des compositions préventives et/ou curatives utiles pour le traitement de maladies neurologiques touchant notamment les neurones du système nerveux central impliquant une hypoxie, une hypoglycémie et/ou une hyperglycémie.

Pour cela, l'invention fournit une composition comprenant au moins un composé de formule (I) : 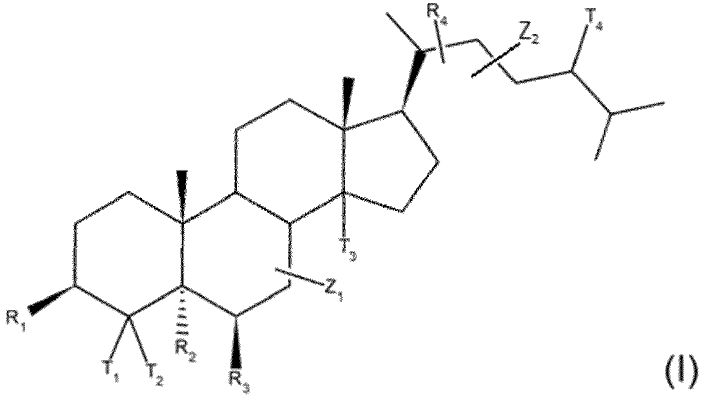 formule dans laquelle
R₁ = OH, F, OCₙH₂ₙ₊₁, R-COO, R-OCOO, RHN-COO ou OPO(OR)₂ avec R = H ou CₙH₂ₙ₊₁ avec n ≤ 16 ;
R₂ = H ou OH ;
R₃ = -NR₅R₆,
R₅ étant H ou -(CH₂)₃NH₂, et
R₆ étant pris dans le groupe formé par -(CH₂)₃NH(CH₂)₄NHR₇ ; -(CH₂)₄NH(CH₂)₃NHR₇ ; -(CH₂)₃NH(CH₂)₄NH(CH₂)₃NHR₇ ; -(CH₂)₃NHR₇ ; -(CH₂)₄NHR₇ avec R₇ = H ou COCH₃ ; -(CH₂)₂-imidazol-4-yl ;-(CH₂)₂-indol-3-yl ; et
R₄ = H ou OH en position 20, 22, 24, 25, 26 ou 27, positionné de façon à créer un centre asymétrique de configuration R ou S ;
Z₁ et Z₂ représentent chacun le nombre de double liaisons entre les atomes de carbone C7 et C8 et C22 et C23 respectivement (soit 0 soit 1); T₁, T₂ et T₃ = H ou CH₃ indépendamment les uns des autres ; T₄ = H, CH₃, C₂H₅ positionné de façon à obtenir un centre asymétrique de configuration R ou S en position 24 ;
et/ou au moins un sel pharmaceutiquement acceptable d'au moins un composé de formule (I),
pour son utilisation dans le traitement d'une pathologie neuronale d'un sujet, ladite pathologie neuronale étant liée à une hypoxie, à une hypoglycémie et/ou à une hyperglycémie affectant des cellules du système nerveux central.

Le composé de formule (I) et défini par : Z₁ = Z₂ = 0 ; R₁ = R₂ = OH ; R₄ = H ; R₅ = H ; R₆ = -*(CH₂)₃-NCOOC(CH₃)₃-(CH₂)₄-NHCOOC(CH₃)₃* ; T₁ = T₂ = T₃ = T₄ = H est appelé DX243BOC, illustré tableau 1.

Le substituant COOC(CH₃)₃ est aussi appelé groupe fonctionnel tert-butoxycarbonyle ou Boc.

Le composé de formule (I) appartient au groupe des stéroïdes. La numérotation des atomes de carbone du composé de formule (I) suit donc la nomenclature définie par l'IUPAC dans Pure & Appl. Chem., Vol.61, No.10, pp.1783-1822,1989. La numérotation des atomes de carbone d'un composé appartenant au groupe des stéroïdes selon l'IUPAC est illustrée ci-dessous :

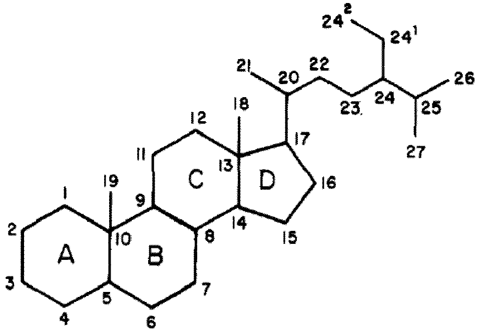

Les méthodes de préparation du composé de formule (I) ont déjà été décrites auparavant, et notamment dans DE MEDINA, P. et al. Synthesis of New Alkylaminooxysterols with Potent Cell Differentiating Activities: Identification of Leads for the Treatment of Cancer and Neurodegenerative Diseases. Journal of Médicinal Chemistry, 52(23), 2009, pp. 7765-7777.

En outre, la composition peut posséder une ou plusieurs caractéristiques suivantes, considérées isolément ou en combinaison.

Selon un mode de réalisation, le composé de formule (I) est défini par Z₂ = 0 ; R₁ = R₂ = OH ; R₄ = H ; R₅ = H ; et T₁ = T₂ = T₃ = T₄ = H.

Selon un mode de réalisation, le composé de formule (I) est défini par Z₁ =0 et R₅ = H.

Selon un mode de réalisation, le composé de formule (I) est défini par R₆ = - (CH₂)₄NH(CH₂)₃NHR₇ avec R₇ = COCH₃.

Selon un mode de réalisation, le composé de formule (I) est défini par R₆ = -(CH₂)₂-imidazol-4-yl.

Selon un mode de réalisation, le composé de formule (I) est défini par R₆ = - (CH₂)₃NH(CH₂)₄NHR₇, -(CH₂)₄NH(CH₂)₃NHR₇, -(CH₂)₃NH(CH₂)₄NH(CH₂)₃NHR₇; ou - (CH₂)₄NHR₇ ; et R₇ = H.

Selon un mode de réalisation, le composé de formule (I) est défini par Z₁ = 1 et R₅ = H.

Selon un mode de réalisation, le composé de formule (I) est défini par :
R₁ = F, OCₙH₂ₙ₊₁, R-COO, R-OCOO, RHN-COO ou OPO(OR)₂ avec R = H ou CₙH₂ₙ₊₁, avec n ≤ 16 ;
R₂= OH ;
R₅ = H ;
R₆ = -(CH₂)₄NH(CH₂)₃NHR₇, -(CH₂)₃NH(CH₂)₄NHR₇, -(CH₂)₃NH(CH₂)₄NH(CH₂)₃NHR₇ ; - (CH₂)₄NHR₇ ;
Z₁ = 0 ou Z1 = 1
Z₂ = 0.

Selon un mode de réalisation, le composé de formule (I) est défini par R₆ = - (CH₂)₃NH(CH₂)₄NHR₇ ; -(CH₂)₄NH(CH₂)₃NHR₇ ; ou -(CH₂)₃NH(CH₂)₄NH(CH₂)₃NHR₇ ; et R₇ = H.

Selon un mode de réalisation, le au moins un composé de formule (I), est défini par Z₁ = Z₂ = 0 ; R₁ = R₂ = OH ; R₄ = H ; R₅ = H ; R₆ = (CH₂)₃NH(CH₂)₄NH₂ ; T₁ = T₂ = T₃ = T₄ = H. Dans ce mode de réalisation le composé est appelé DX243. Les résultats obtenus avec ce composé sont particulièrement intéressants. En effet, on observe un effet curatif de ce composé contre le phénomène pathophysiologique d'hypoxie, d'hypoglycémie et/ou d' hyperglycémie même à de très faibles concentrations.

Selon un mode de réalisation, le au moins un composé de formule (I), est défini par Z₁ = Z₂ = 0 ; R₁ = R₄ = H ; R₂ = OH ; R₅ = H ; R₆ = (CH₂)₄NH(CH₂)₃NH₂ ; T₁ = T₂ = T₃ = T₄ = H. Dans ce mode de réalisation le composé est appelé DX245. Les résultats obtenus avec ce composé sont particulièrement intéressants. En effet, on observe un effet curatif de ce composé contre le phénomène pathophysiologique d'hypoxie, d'hypoglycémie et/ou d' hyperglycémie supérieur à celui du DX243.

Selon un mode de réalisation, le au moins un composé de formule (I), est défini par Z₁ = 1 ; Z₂ = 0 ; R₁ = R₄ = H ; R₂ = OH ; R₅ = H ; R₆ = (CH₂)₃NH(CH₂)₄NH₂ ; T₁ = T₂ = T₃ = T₄ = H. Dans ce mode de réalisation le composé est appelé DX242. Les résultats obtenus avec ce composé sont particulièrement intéressants. En effet, on observe un effet curatif de ce composé contre le phénomène pathophysiologique d'hypoxie, d'hypoglycémie et/ou d' hyperglycémie supérieur à celui du DX243.

Selon un mode de réalisation, le au moins un composé de formule (I), est défini par Z₁ = 1 ; Z₂ = 0 ; R₁ = R₄ = H ; R₂ = OH ; R₅ = H ; R₆ = (CH₂)₄NH(CH₂)₃NH₂ ; T₁ = T₂ = T₃ = T₄ = H. Dans ce mode de réalisation le composé est appelé DX244. Les résultats obtenus avec ce composé sont particulièrement intéressants. En effet, on observe un effet curatif de ce composé contre le phénomène pathophysiologique d'hypoxie, d'hypoglycémie et/ou d' hyperglycémie supérieur à celui du DX243.

Selon un mode de réalisation, la pathologie neuronale du système nerveux central est prise dans le groupe constitué des traumatismes cérébraux et des accidents vasculaires cérébraux.

Selon un mode de réalisation, la pathologie neuronale du système nerveux central est une lésion cérébrale due à une ischémie.

Selon un mode de réalisation, la pathologie neuronale du système nerveux central est une lésion cérébrale due à une insuffisance respiratoire. On entend par insuffisance respiratoire l'insuffisance respiratoire obstructive et l'insuffisance respiratoire restrictive. Cette dernière pouvant provenir par exemple d'une infection pulmonaire d'origine bactérienne ou virale, par exemple suite à une infection par un coronavirus.

Selon un mode de réalisation, l'hypoglycémie est due à un diabète.

Selon un mode de réalisation, l'hyperglycémie est due à un diabète.

Selon un mode de réalisation, l'invention fournit aussi une composition pour son utilisation dans le traitement d'une pathologie neuronale d'un sujet, ladite pathologie neuronale étant liée à une hypoxie, à une hypoglycémie et/ou à une hyperglycémie affectant des cellules du système nerveux central, ladite composition étant sous forme de solution aqueuse et présentant une concentration de composé de formule (I) comprise entre 1 pmol.L⁻¹ et 1 mmol.L⁻¹, préférentiellement entre 10 pmol.L⁻¹ et 0,1 mmol.L⁻¹, plus préférentiellement entre 0,1 nmol.L⁻¹ et 1 µmol.L⁻¹. Par exemple, la concentration de composé de formule (I) est comprise entre 1 nmol.L⁻¹ et 150 nmol.L⁻¹.

### Brève description des figures

L'invention sera mieux comprise, et d'autres buts, détails, caractéristiques et avantages de celle-ci apparaîtront plus clairement au cours de la description suivante de plusieurs modes de réalisation particuliers de l'invention, donnés uniquement à titre illustratif et non limitatif, en référence aux dessins annexés.
[fig. 1] La figure 1 représente schématiquement un protocole d'étude de l'efficacité de composés de formule (I), en particulier du DX243, sur un modèle in vitro d'ischémie.
[fig. 2] La figure 2 illustre les résultats d'un test immunocytochimique afin d'évaluer la survie neuronale dans un modèle in vitro d'ischémie/reperfusion en présence de DX243.
[fig. 3] La figure 3 illustre les résultats d'un test d'évaluation de survie neuronale MTT dans un modèle in vitro d'ischémie/reperfusion en présence de DX243.
[fig. 4] La figure 4 illustre les résultats d'un test d'évaluation de survie neuronale via le bleu de trypan dans un modèle in vitro d'ischémie/reperfusion en présence de DX243.
[fig. 5] La figure 5 illustre les résultats d'un test d'évaluation de survie neuronale via le bleu de trypan dans un modèle in vitro d'ischémie/reperfusion en présence de composés formule (I) du tableau 1, autre que le DX243.
[fig. 6] La figure 6 illustre les résultats d'un test d'évaluation de survie neuronale MTT dans un modèle in vitro d'ischémie/reperfusion en présence des composés du tableau 1.
[fig. 7] La figure 7 illustre les résultats d'un second test d'évaluation de survie neuronale via le bleu de trypan dans un modèle in vitro d'ischémie/reperfusion en présence des composés de formule (I) du tableau 1.
[fig. 8] La figure 8 illustre les résultats du test in vivo d'AVC chez la souris afin d'évaluer la survie neuronale après avoir subi un AVC suivi d'une reperfusion avec traitement au DX243.
[fig. 9] La figure 9 est un ensemble de graphique illustrant les résultats du test in vivo d'AVC chez la souris afin d'évaluer la survie neuronale après avoir subi un AVC suivi d'une reperfusion avec traitement au DX243.

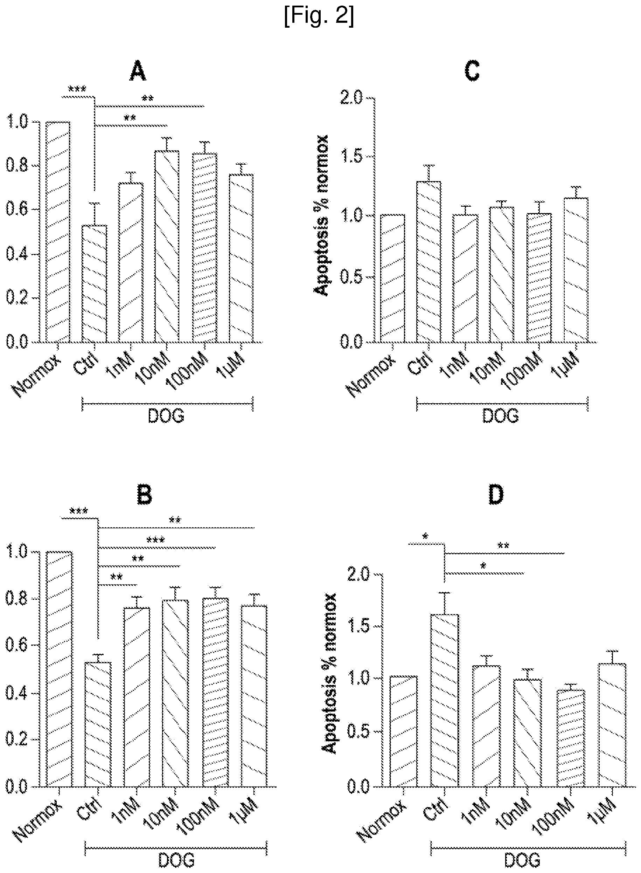

Sur les figures 2 à 4, 6 et 7, les étoiles indiquent la puissance statistique des résultats. Une étoile indique qu'il est certain à 95% que les résultats ne sont pas dus au hasard. La présence de 2 étoiles signifie qu'il est certains à 99% que les résultats ne sont pas dus au hasard, et la présence de 3 étoiles indiquent qu'il est certain à 99,9% que les résultats ne sont pas dus au hasard.

### Description des modes de réalisation

On va décrire ci-dessous plusieurs protocoles expérimentaux qui mettent en évidence l'effet protecteur des composés de formule (I) indiqués dans le tableau 1, dont le composé DX243, contre l'hypoxie et l'hypoglycémie.

Les concentrations ou molarités des composés sont exprimées en mole par litre dont le symbole est mol.L⁻¹ ou M.

### Exemple 1 : obtention des neurones corticaux

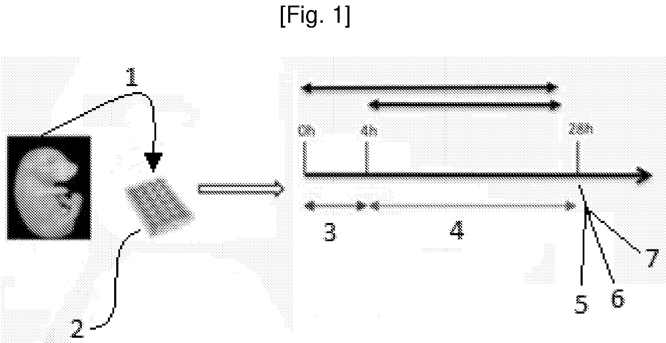

En référence à la figure 1, la première étape du protocole d'étude de l'effet neuroprotecteur des composés de formule (I), en particulier ceux indiquées dans le tableau 1, consiste à obtenir une culture primaire de neurones corticaux à partir de cellules prélevées sur des cerveaux d'embryons de souris de type sauvage mises dans des conditions de culture adéquates (étape 1). Plus précisément, les cellules prélevées sont crues dans un milieu Neurobasal^{™} Medium (Ref. 21103049 ThermoFisher Scientific) auquel de la L-Glutamine et du B27 supplement 50X (Ref. 17504044 ThermoFisher Scientific) ont été ajoutés (étape 2). Les neurones issus de la culture primaire sont ensuite isolés et purifiés. Il est à noter que ce sont les conditions de culture en elles-mêmes qui permettent d'obtenir une culture purifiée en neurones à partir de la dissociation des cortex embryonnaires. Une déprivation en oxygène et en glucose (DOG) est alors réalisée (étape 3) sur ces neurones afin de mimer le plus fidèlement possible ce qu'il se passe in vivo lors d'un accident vasculaire cérébral c'est-à-dire une diminution de l'apport en oxygène et en glucose dû à une diminution de la perfusion sanguine pour les cellules. Pour réaliser cette déprivation en oxygène et en glucose mimant l'ischémie, les neurones sont placés pendant 4h dans un incubateur dont l'atmosphère a une teneur en oxygène d'environ 1% et en remplaçant le milieu de culture par un milieu sans glucose (étape 3). Comme le modèle d'ischémie est un modèle dit d'ischémie/reperfusion, les neurones sont ensuite placés dans un milieu contenant du glucose dans des conditions normoxiques pendant 24h (reperfusion) (étape 4) suite à l'étape d'ischémie. Le groupe A de neurones est un groupe de neurones ayant été traité avec une solution de composé de formule (I) pendant la DOG et la reperfusion, c'est-à-dire qu'une molécule du tableau 1 est ajoutée dans le milieu sans glucose dès que la déprivation en glucose et en oxygène commence (étape 3). La concentration du composé de formule (I) nommé DX243 est comprise entre 1 nmol.L⁻¹ et 1 pmol.L⁻¹ dans le milieu sans glucose. La concentration des autres molécules du tableau 1, est de 100 nmol.L⁻¹ dans le milieu sans glucose. Le groupe B de neurones est un groupe de neurones ayant été traité avec une solution de DX243 pendant la reperfusion seulement (étape 4), pour se rapprocher de ce qui se passe cliniquement, à savoir le traitement possible uniquement après plusieurs heures avec un traitement conventionnel. La solution de DX243 est ajoutée au milieu sans glucose dès que la reperfusion commence (étape 4). La concentration de DX243 est comprise entre 1 nmol.L⁻¹ et 1 pmol.L⁻¹ dans le milieu sans glucose.

Le contrôle négatif consiste en un groupe de neurones subissant la DOG (étape 3) suivie de la reperfusion (étape 4) sans DX243 présent. Ce groupe de neurones est dénommé « Ctrl » dans les figures 2 à 4. Le contrôle normal consiste en un groupe de neurones issus de la culture primaire placé en conditions normoxiques pendant 28h. Le contrôle normal est dénommé « normox » dans les figures 2 à 4. Le contrôle positif consiste en un groupe de neurones issus de la culture primaire ayant subi la DOG pendant 4h (étape 3) en présence de roscovitine, un inhibiteur du cycle cellulaire, entrainant une protection neuronale, suivie de la reperfusion pendant 24h (étape 4). Le contrôle positif est dénommé « ROSCO » dans les figures 2 à 4.

A la fin de la reperfusion (étape 4), 24 h après la DOG, la survie des neurones est évaluée à l'aide de trois tests différents : un test immunocytochimique 5, un test MTT (bromure de 3-(4,5-dimethylthiazol-2-yl)-2,5-diphenyl tetrazolium) 6 et un test basé sur l'intégrité des membranes cellulaire via l'utilisation du bleu de triptan 7. Les résultats sont alors comparés aux résultats des trois tests de survie des groupes de contrôle.

### Exemple 2 : Test immunocytochimique de survie neuronale dans le cas de l'utilisation du DX243

La figure 2 présente les résultats du premier test de survie neuronale dans le cas de l'utilisation du DX243. Les graphes A et C sont une représentation des résultats obtenus pour le groupe A, l'ordonnée représente la proportion de cellules vivantes par rapport au groupe Normox. Les graphes B et D sont une représentation des résultats obtenus pour le groupe B, l'ordonnée représente la proportion de cellules en apoptoses par rapport au groupe Normox. Ce premier test est un test immunocytochimique qui permet de mettre en évidence les cellules vivantes et les cellules mortes.

Une première série d'images est réalisée avec un marquage chimique par fluorescence. Ce marquage est effectué avec du DAPI (4',6-diamidino-2-phénylindole) qui est capable de se lier fortement aux bases adénine (A) et thymine (T) de l'ADN. Il permet de détecter les cellules vivantes. Une deuxième série d'images est réalisée avec un marquage par fluorescence utilisant l'anticorps CC3. L'anticorps CC3 permet de détecter la caspase 3 activée, et donc les cellules en état d'apoptose.

Pour visualiser la proportion de neurones sains parmi les différentes conditions de cultures, l'anticorps TUJ1 est utilisé. TUJ1 réagit avec la bêta-tubuline III, une protéine structurale composant la tubuline et spécifique aux neurones. La bêta-tubuline III est largement utilisée comme marqueur pour distinguer les neurones d'autres types de cellules.

A partir de ces images, la proportion de cellules vivantes (cell survival) a été calculée en déterminant le rapport du nombre de cellules vivantes sur le nombre total de cellules. La proportion de cellules en apoptose a été réalisée à l'aide du rapport entre le nombre de cellules détectées par l'anticorps CC3 sur le nombre total de cellules. Les valeurs obtenues ont été rapportées à celles obtenues par le groupe normox.

On observe que la DOG induit une diminution de la proportion de cellules vivantes sur le groupe Ctrl sur les graphes A et B de la figure 2, et une augmentation de la proportion de cellules en apoptose sur le groupe Ctrl sur les graphes C et D de la figure 2. En revanche, aussi bien un traitement avec du DX243 pendant la DOG et la reperfusion qu'un traitement avec du DX243 uniquement pendant la reperfusion, protège partiellement de la mort neuronale. En effet, on observe une augmentation de la proportion de cellules vivantes et une diminution de la proportion de cellules en apoptose suite au traitement au DX243 en comparaison avec le groupe Ctrl. Une concentration en DX243 située entre 1 nmol.L⁻¹ et 100 nmol.L⁻¹ correspond à une gamme de concentration idéale pour maximiser les effets neuroprotecteurs.

De plus, il apparaît qu'un traitement post-DOG pendant 24h au DX243 protège encore plus efficacement les neurones.

### Exemple 3 : Test d'évaluation de survie neuronale MTT dans le cas de l'utilisation du DX243

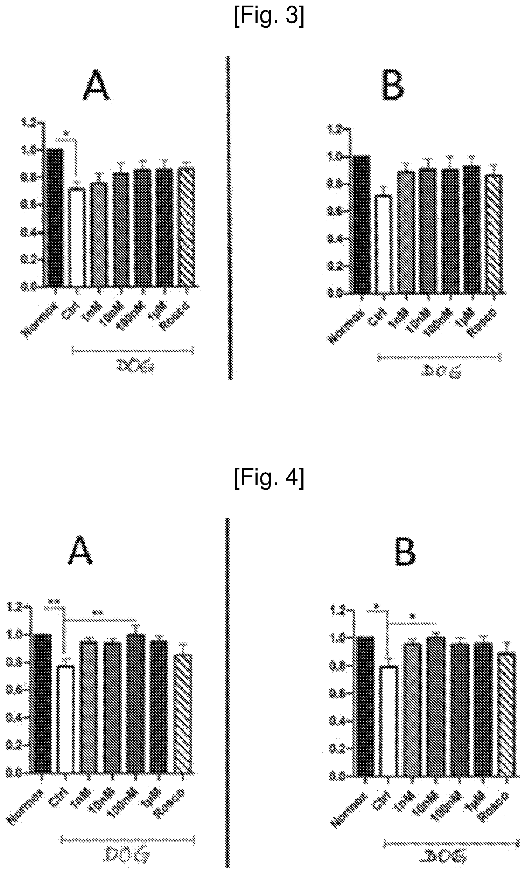

Pour confirmer les résultats obtenus avec le DX243, un test basé sur l'activité métabolique des neurones a été réalisé pour évaluer d'une autre façon la survie neuronale. Ce test est basé sur l'utilisation du sel de tétrazolium MTT (bromure de 3-(4,5-dimethylthiazol-2-yl)-2,5-diphenyl tetrazolium). Le tétrazolium est réduit par la succinate déshydrogénase mitochondriale des cellules vivantes actives, en formazan, précipité de couleur violette. La quantité de précipité formée est proportionnelle à la quantité de cellules vivantes mais également à l'activité métabolique de chaque cellule. Ainsi, un simple dosage de la densité optique à 550 nm par spectroscopie permet de connaître la quantité relative de cellules vivantes et actives métaboliquement. Etant donné que plus le nombre de cellules vivante est important, plus l'intensité colorimétrique est importante, l'intensité colorimétrique a dès lors été quantifiée et rapportée à l'intensité observée en conditions normoxiques. Les résultats obtenus ont été rapportés dans le graphe A de la figure 3 pour le groupe A et dans le graphe B de la figure 3 pour le groupe B.

Sur les graphes A et B de la figure 3, on observe que la DOG entraine une diminution marquée de cellules métaboliquement actives dans le groupe Ctrl. En revanche, le traitement des neurones avec du DX243 protège partiellement de cette diminution de survie suite à la DOG. Cet effet est d'autant plus marqué pour le groupe de neurones ayant bénéficié du DX243 que pendant la reperfusion de 24h comme le montre le graphe B de la figure 3. Sur les deux graphes, un traitement comprenant une concentration de DX243 comprise entre 10 nmol.L⁻¹ et 100 nmol.L⁻¹ semble le plus efficace.

### Exemple 4 : Test d'évaluation de survie neuronale via le bleu de trypan dans le cas de l'utilisation du DX243

Un troisième test de survie cellulaire dans le cas de l'utilisation du DX243, basé sur l'intégrité des membranes cellulaire, laquelle est rompue chez les cellules mortes, a été réalisé. Ce dernier test utilise le bleu de trypan qui colorera les cellules mortes en bleu. Le calcul du pourcentage de cellules vivantes a dès lors été effectué en comptant la proportion de cellules bleues et non-bleues, et rapporté au pourcentage de cellules vivantes observé dans le groupe normox. Les résultats obtenus ont été rapportés dans le graphe A de la figure 4 pour le groupe A et dans le graphe B de la figure 4 pour le groupe B.

On observe que la DOG induit une diminution significative du nombre de cellules ayant conservé l'intégrité de leur membrane que ce soit sur le graphe A de la figure 4 ou sur le graphe B de la figure 4. Le traitement des neurones avec du DX243 restore le pourcentage de cellules vivantes à des niveaux proches de celui observé dans le groupe normox, principalement avec des concentrations entre 1 nmol.L⁻¹ et 100 nmol.L⁻¹.

Le DX243 est donc efficace à des concentrations comprises entre 1 nmol.L⁻¹ et 1 µmol.L⁻¹. Pour les concentrations de DX243 comprises entre 10 nmol.L⁻¹ et 100 nmol.L⁻¹, les résultats sont plus stables et l'effet protecteur du DX243 est statistiquement plus importants. Cela peut s'expliquer par la fragilité des cultures primaires en dehors de leur environnement naturel.

### Exemple 5 : Test d'évaluation de survie neuronale via le bleu de trypan dans le cas de l'utilisation des molécules du tableau I

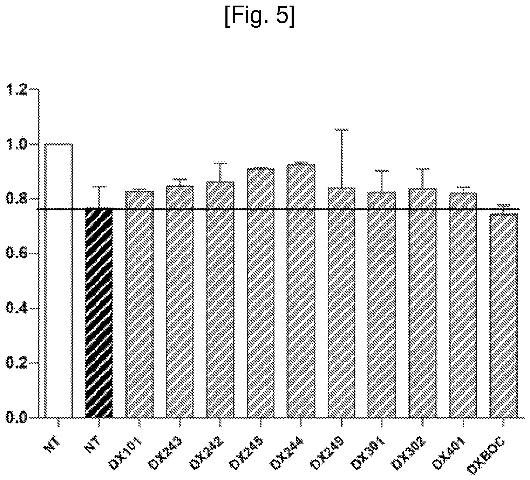

Pour toutes les molécules du tableau I autre que le DX243 utilisées sur le groupe A, le troisième test de survie cellulaire a été réalisé. Les résultats obtenus ont été rapportés sur le graphe de la figure 5 en y incluant les résultats obtenus avec le DX243. Le sigle NT signifie non traité. La barre non hachurée NT correspond à un groupe de neurones issus de la culture primaire placé en conditions normoxiques pendant 28h. Les barres hachurées représentent des groupes de neurones issus de la culture primaire ayant subie une DOG et la reperfusion soit sans molécules du tableau 1 (barre hachurée nommée NT) soit en présence d'une molécule du tableau 1. Le calcul du pourcentage de cellules vivantes a été effectué en comptant la proportion de cellules bleues et non-bleues, et rapporté au pourcentage de cellules vivantes observé dans le groupe NT en condition de normoxie, c'est-à-dire le groupe NT n'ayant pas subi la DOG (non représenté).

Pour tous les composés testés à l'exception du DX243BOC, le taux de survie des groupes de neurones est supérieur à celui des groupes de neurones NT ayant subie la DOG et la reperfusion (barre hachurée NT), ils présentent donc un effet neuroprotecteur. En particulier, il est à noter que le DX245, le DX244 et le DX242 ont un taux de survie supérieur à celui du DX243.

Le taux de survie du groupe traité avec DX243BOC est quant à lui inférieur à celui du groupe traité avec le DX243 et à celui du groupe NT ayant subi la DOG et la reperfusion démontrant ainsi l'importance du groupe R₃ dans l'activité du composé de formule (I).

### Exemple 6 : Test d'évaluation de survie neuronale MTT dans le cas de l'utilisation des molécules du tableau I

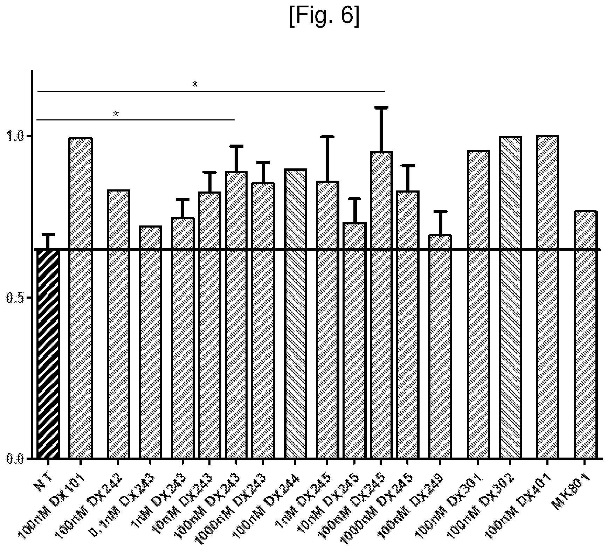

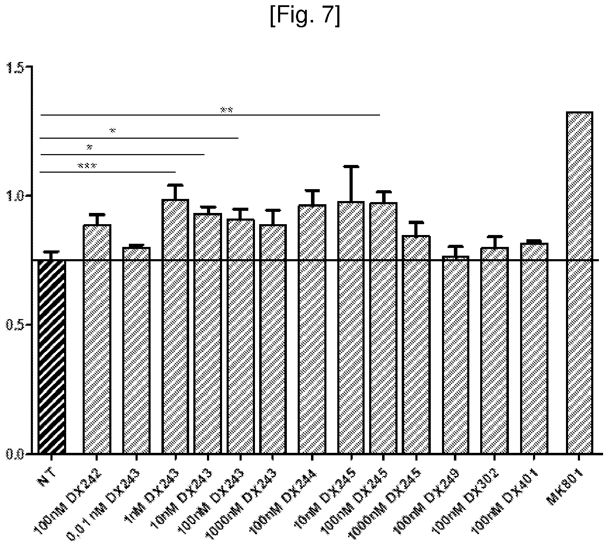

Sur le graphe figure 6, un test similaire au tableau graphe A de la figure 3 a été réalisé. C'est-à-dire un test MTT afin d'évaluer l'effet neuroprotecteur des molécules après avoir subi un traitement d'une durée de 4h DOG puis 24h de reperfusion. Le test est également basé sur le protocole d'obtention des neurones corticaux de l'exemple 1 énoncé ci-dessus. A la différence de l'exemple figure 3 A, le contrôle positif Roscovitine a été remplacé par un contrôle positif Dizocilpine (MK801). Ce contrôle positif consiste également en un groupe de neurones issus de la culture primaire ayant subi la DOG pendant 4h en présence de Dizocilpine, suivie de la reperfusion pendant 24h. Le contrôle positif est dénommé « MK801 » dans les figures 6 et 7. En ordonnée des figures 6 et 7, il est illustré en pourcentage le rapport des cellules vivantes traitées au regard des cellules vivantes en condition de normoxie, non représenté dans le graphique car égal à 100%. MK801 est un antagoniste non compétitif du récepteur N-méthyl-D-aspartate (NMDA) et est connu pour avoir un effet neuroprotecteur. Tous les composés du tableau 1 ont été testés. Il est observé figure 6 une protection neuronale significative avec un traitement à 100nM de DX243 et DX245. Un effet neuroprotecteur est observé avec ce test pour DX101, DX242, DX244, DX301, DX302, DX401, DX249.

Pour tous les composés testés dans cet exemple, le taux de survie des groupes de neurones est supérieur à celui des groupes de neurones NT, ils présentent donc un effet neuroprotecteur.

### Exemple 7 : Test d'évaluation de survie neuronale via le bleu de trypan dans le cas de l'utilisation des molécules du tableau I

Un nouveau test de survie cellulaire des différents composés du tableau 1 a été réalisé, les résultats sont présentés figure 7. Ce test est similaire à celui réalisé figure 4. Il est basé sur l'intégrité des membranes cellulaire, laquelle est rompue chez les cellules mortes. Ce test utilise le bleu de trypan qui colorera les cellules mortes en bleu. Il est réalisé de manière similaire au test de la figure 4 A, c'est-à-dire basé sur le protocole d'obtention des neurones corticaux de l'exemple 1 énoncé ci-dessus. A la différence de l'exemple figure 4 A, le contrôle positif Roscovitine a été remplacé par le contrôle positif Dizocilpine (MK801). Ce test a pour but d'évaluer l'effet neuroprotecteur des molécules après avoir subi un traitement d'une durée de 4h DOG puis 24h de reperfusion, soit un total de 28 h d'expériences. Tous les composés du tableau 1 ont été testés plusieurs fois. En outre, DX243 et DX245 ont été testés dans différentes quantités indiquées sur la figure 7.

A l'issu de cette expérience, il est observé une protection neuronale significative avec un traitement à 100 nM de DX245 et pour les concentrations allant de 1 à 100 nM de DX243. Il est également observé un effet neuroprotecteur avec ce test pour le 10 nM de DX245, 1000 nM de DX245, 1000 nM de DX243, DX242 et 244.

Il est donc attendu que dans une solution aqueuse pharmaceutiquement acceptable comprenant le composé de formule (I), une concentration en composé de formule (I) comprise entre 1 pmol.L⁻¹ et 1 mmol.L⁻¹, préférentiellement entre 10 pmol.L⁻¹ et 0,1 mmol.L^{- 1}, plus préférentiellement entre 0,1 nmol.L⁻¹ et 1 pmol.L⁻¹ soit efficace.

### Exemple 8 : étude de l'effet neuroprotecteur in vivo dans un modèle murin

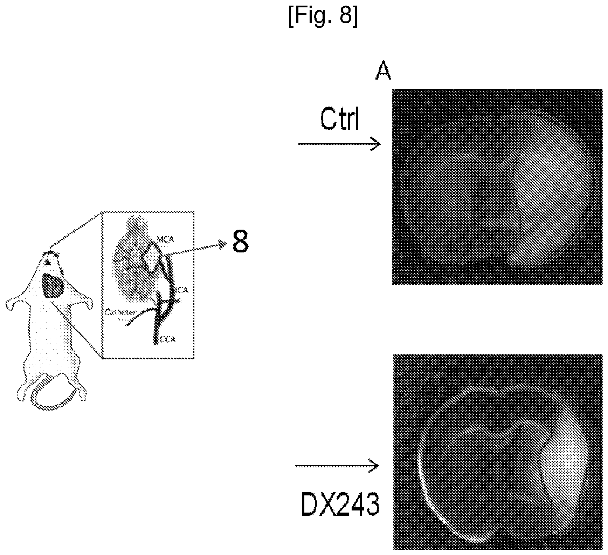

Une étude a été réalisée in vivo afin de démontrer l'effet neuroprotecteur de la molécule DX243 dans un modèle murin mâle d'environ 20,5 grammes (g) à 22g d'AVC nommé *Middle Cérébral Artery Occlusion* (MCAO), représenté figure 8. Ce modèle in vivo classiquement utilisé pour étudier l'effet de molécules sur l'AVC consiste en l'installation d'un cathéter au niveau du cortex cingulaire antérieur (CCA), à y insérer un filament au niveau de l'artère carotide commune (CCA-common carotide artery) et de pousser le filament à travers l'artère carotide commune, passant par l'artère carotide interne (ICA) jusqu'au niveau de l'artère cérébrale moyenne (MCA) entrainant une diminution importante (>80%) du flux sanguin au niveau du territoire cérébral 8 irrigué par cette artère (Estelle ROUSSELET et al, Modèle murin d'MCAO intraluminale: Évaluation infarctus cérébral par coloration au violet de crésyl, Journal of Visualized Experiments and Carl Zeiss). A la suite d'une durée de 1h, le filament est retiré pour permettre la reperfusion et l'ajout du traitement au DX243. Le DX243 injecté est dilué dans une solution aqueuse et est concentré à 50mg/Kg.

Les conséquences sont observées 24h après l'insertion du filament jusqu'au niveau de l'artère cérébrale moyenne, qu'elles soient tissulaires (chlorure de triphényltétrazolium (TTC) marquant en rouge le tissu vivant) ou comportementales (neuroscore). Le neuroscore est une évaluation des déficits neurologiques de la souris permettant l'évaluation de la réussite de MCAO. L'échelle utilisée comporte 5 points :
0 : état normal
1 : un léger comportement d'encerclement avec ou sans rotation incohérente lorsqu'il est saisi par la queue, <50% de tentatives de rotation vers le côté controlatéral.
2 : cerclage léger et constant, >50% de tentatives de rotation vers le côté controlatéral.
3 : effectue des cercles réguliers, forts et immédiats, la souris maintient une position de rotation pendant plus de 1 à 2 secondes, son nez atteignant presque sa queue.
4 : rotation sévère progressant en tonneau, perte du réflexe de marche ou de redressement.
5 : comateux ou moribond.

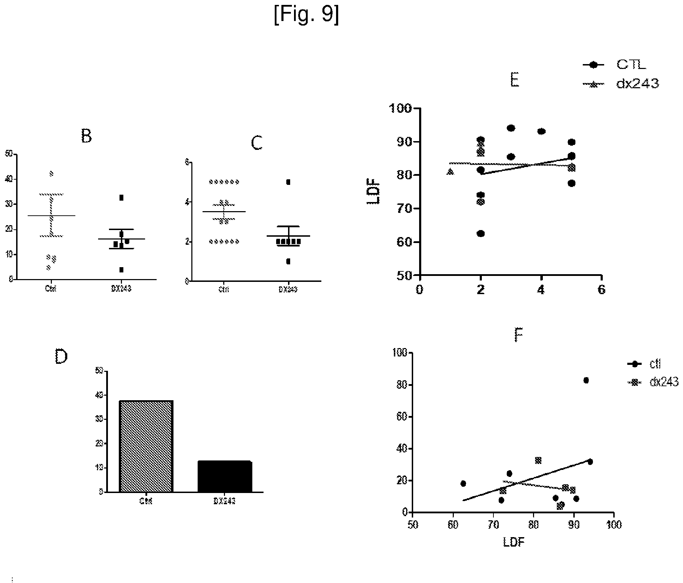

Les résultats in vivo de l'effet neuroprotecteur du DX243 sont présentés figures 8 et 9. On observe figure 8 (A) deux images en coloration au TTC montrant en blanc la surface de tissu mort et représentant la diminution de la surface infarcie chez les souris traitées au DX243. Le graphique B de la figure 9 représente la diminution du pourcentage de surface infarcie entre souris traitées ou non-traitées (Ctrl), en ordonnée est indiquée le pourcentage de la zone infarcie au regard de la taille totale du cerveau de la souris. Le graphique C illustre l'amélioration comportementale (neuroscore) chez les souris traitées. Le graphique D représente la diminution du pourcentage de souris morte chez les souris traitées au DX243. Les graphiques E et F représentent la disparition de corrélation entre diminution du flux sanguin et conséquence histologiques et comportementale suite au traitement au DX 243. La diminution du flux sanguin a été mesurée par *Laser Doppler Flowmetry* (LDF). Il a été mis en évidence figure 9 qu'un traitement au DX243 permettait d'améliorer en 24h les conséquences d'un AVC chez la souris. En effet, chez le groupe de souris traitées au DX243, il est observé sur le graphe D une diminution du pourcentage de souris mortes. Il est également illustré sur le graphe B une diminution de la taille de la lésion (zone infarcie) et dans le graphe C une diminution des déficits comportementaux (neuroscore) chez les souris vivantes. En outre, il est observé via un laser doppler dont les résultats sont présentés graphes E et F que plus la diminution du flux sanguin était importante, plus les déficits étaient importants. Par contre, chez les souris traitées, il ne semble plus y avoir de corrélations entre la diminution du flux sanguin et les conséquences de l'AVC. Ces résultats in vivo démontrent l'effet neuroprotecteur observé des molécules selon l'invention.

L'ensemble de ces résultats indique que les composés DX101, DX243, DX242, DX245, DX244, DX249, DX301, DX302, DX401 du tableau 1 possèdent un effet neuroprotecteur contre l'ischémie cérébrale, et donc contre les pathologies neuronales liées à une hypoxie, à une hypoglycémie et/ou à une hyperglycémie telles que les traumatismes cérébraux et les accidents vasculaires cérébraux.

Bien que les expériences aient été réalisées avec dix composés de formule (I) différents, il est bien évident que des résultats similaires sont attendus pour les composés de formule (I) autre que ceux du tableau 1.

En outre, dans le cadre d'une hypoxie seule ou d'une hypoglycémie seule, une composante mort neuronale apoptotique est observée, comme décrit dans KATO, et al. (« Recurrent short-term hypoglycemia and hyperglycemia induce apoptosis and oxidative stress via the ER stress response in immortalized adult mouse Schwann (INMS32) cells ». Neuroscience Research, 13 novembre 2018, retrieved from < https://www.sciencedirect.com/science/article/pii/S0168010218304371?via%3Dihub> <doi : https://doi.org/10.1016/j.neures.2018.11.004>), dans Sendoel, A., et al. (« Apoptotic Cell Death Under Hypoxia ». Physiology, 29, pp.168-176, 2014), ou encore dans Xu, Y., et al. (« Protective effect of lithium chloride agains hypoglycemia-induced apoptosis in neuronalPC12 cell ». Neuroscience, 330, 25 août 2016, pp.100-108). Ainsi, les résultats observés ci-dessus dans le modèle d'ischémie/reperfusion montre une protection due aux composés de formule (I) face à cette composante apoptotique. De plus, une hypoglycémie est capable d'induire un stress oxydatif dans des cellules neuronales, stress oxydatif présent dans le cadre d'une déprivation en oxygène et en glucose et qui est une composante majeure de l'induction de la mort neuronale. En conséquence, des mécanismes communs induisant la mort neuronale existent dans le cadre d'une mort neuronale induite par hypoglycémie et hypoxie, par hypoglycémie seule et par hypoxie seule, tels que l'apoptose. En conclusion l'effet neuroprotecteur des composés de formules (I) selon l'invention s'étend aux cas d'hypoxie seule ou d'hypoglycémie seule.

L'usage du verbe « comporter », « comprendre » ou « inclure » et de ses formes conjuguées n'exclut pas la présence d'autres éléments ou d'autres étapes que ceux énoncés dans une revendication.

Dans les revendications, tout signe de référence entre parenthèses ne saurait être interprété comme une limitation de la revendication.

**Tableau 1 :**

| Nom | Structure | Nom | Structure |
|---|---|---|---|
| **DX101** | 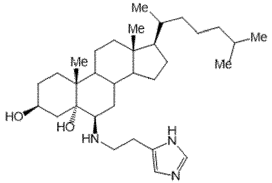 | **DX249** | 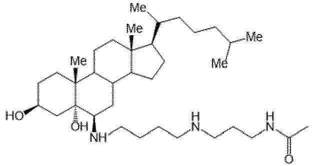 |
| **DX243** | 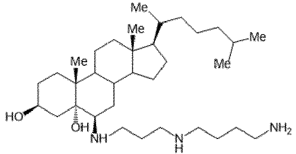 | **DX301** | 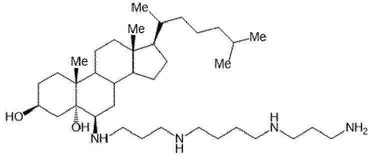 |
| **DX242** | 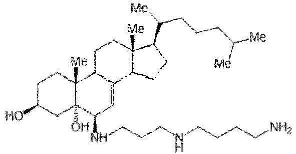 | **DX302** | 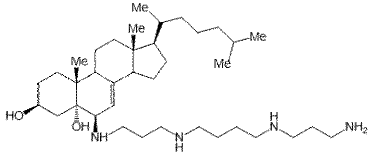 |
| **DX245** | 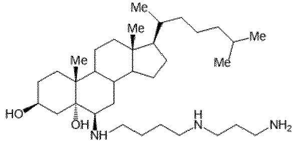 | **DX401** | 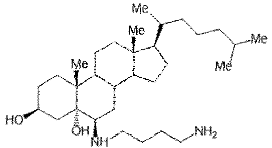 |
| **DX244** | 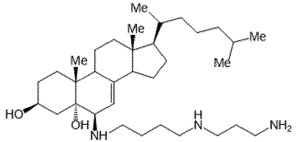 | **DX243BOC** | 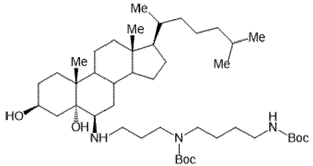 |

## Revendications

1. Composition comprenant au moins un composé de formule (I) : 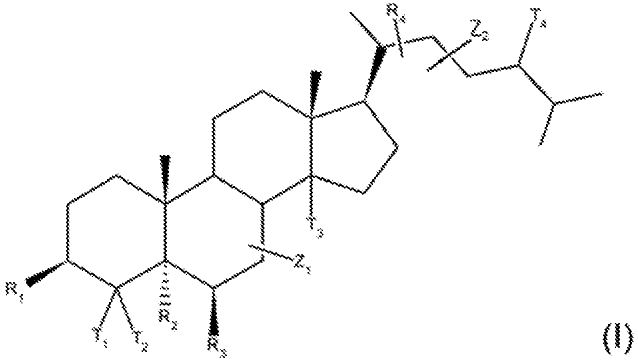 formule dans laquelle
R₁ = OH;
R₂ = OH ;
R₃ = -NR₅R₆,
R₅ étant H, et
R₆ étant pris dans le groupe formé par -(CH₂)₃NH(CH₂)₄NHR₇ ; - (CH₂)₄NH(CH₂)₃NHR₇ ; -(CH₂)₃NH(CH₂)₄NH(CH₂)₃NHR₇ ; -(CH₂)₃NHR₇ ; - (CH₂)₄NHR₇ avec R₇ = H ou COCH₃ ; -(CH₂)₂-imidazol-4-yl ;et -(CH₂)₂-indol-3-yl ; et
R₄ = H en position 20, 22, 24, 25, 26 ou 27, positionné de façon à créer un centre asymétrique de configuration R ou S ;
Z₁ et Z₂ représentent chacun le nombre de double liaisons entre les atomes de carbone C7 et C8 et C22 et C23 respectivement ; Z₁ est soit 0 soit 1 ; Z₂ = 0 ; T₁=T₂=T₃=T₄=H, T₄ étant positionné de façon à obtenir un centre asymétrique de configuration R ou S en position 24 ;
et/ou au moins un sel pharmaceutiquement acceptable d'au moins un composé de formule (I),
pour son utilisation dans le traitement d'une pathologie neuronale d'un sujet, ladite pathologie neuronale étant liée à une hypoxie, à une hypoglycémie et/ou à une hyperglycémie affectant des cellules du système nerveux central.

2. Composition pour utilisation selon la revendication 1, dans laquelle le composé de formule (I) est défini par Z₁ =0.

3. Composition pour utilisation selon la revendication 2, dans laquelle le composé de formule (I) est défini par R₆ = -(CH₂)₄NH(CH₂)₃NHR₇ avec R₇ = COCH₃.

4. Composition pour utilisation selon la revendication 2, dans laquelle le composé de formule (I) est défini par R₆ = -(CH₂)₂-imidazol-4-yl.

5. Composition pour utilisation selon la revendication 2, dans laquelle le composé de formule (I) est défini par R₆ = -(CH₂)₃NH(CH₂)₄NHR₇, - (CH₂)₄NH(CH₂)₃NHR₇, -(CH₂)₃NH(CH₂)₄NH(CH₂)₃NHR₇ ; ou -(CH₂)₄NHR₇ ; et R₇ = H.

6. Composition pour utilisation selon la revendication 1, dans laquelle le composé de formule (I) est défini par Z₁ = 1.

7. Composition pour utilisation selon la revendication 6, dans laquelle le composé de formule (I) est défini par R₆ = -(CH₂)₃NH(CH₂)₄NHR₇ ; - (CH₂)₄NH(CH₂)₃NHR₇ ; ou -(CH₂)₃NH(CH₂)₄NH(CH₂)₃NHR₇ ; et R₇ = H.

8. Composition comprenant au moins un composé de formule (I) : 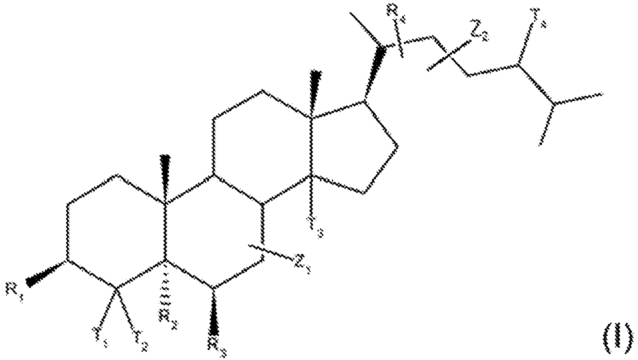 formule dans laquelle
R₁ = F, OCₙH₂ₙ₊₁, R-COO, R-OCOO, RHN-COO ou OPO(OR)₂ avec R = H ou CₙH₂ₙ₊₁, avec n ≤ 16 ;
R₂ = OH
R₃ = -NR₅R₆,
R₄ = H ou OH en position 20, 22, 24, 25, 26 ou 27, positionné de façon à créer un centre asymétrique de configuration R ou S ;
R₅ = H ;
R₆ étant pris dans le groupe formé par -(CH₂)₄NH(CH₂)₃NHR₇, - (CH₂)₃NH(CH₂)₄NHR₇, -(CH₂)₃NH(CH₂)₄NH(CH₂)₃NHR₇ ; -(CH₂)₄NHR₇ ; avec R₇ = H ou COCH₃ ;
-(CH₂)₂-imidazol-4-yl ; et -(CH₂)₂-indol-3-yl ;
Z₁ et Z₂ représentent chacun le nombre de double liaisons entre les atomes de carbone C7 et C8 et C22 et C23 respectivement,
Z₁ = 0 ou Z1 = 1
Z₂ = 0 ;
T₁, T₂, T₃ et T₄ = H, T₄ étant positionné de façon à obtenir un centre asymétrique de configuration R ou S en position 24 ;
et/ou au moins un sel pharmaceutiquement acceptable d'au moins un composé de formule (I),
pour son utilisation dans le traitement d'une pathologie neuronale d'un sujet, ladite pathologie neuronale étant liée à une hypoxie, à une hypoglycémie et/ou à une hyperglycémie affectant des cellules du système nerveux central.

9. Composition pour utilisation selon l'une des revendications 1 à 8, dans laquelle la pathologie neuronale du système nerveux central est choisie parmi le groupe constitué des traumatismes cérébraux et des accidents vasculaires cérébraux.

10. Composition pour utilisation selon l'une des revendications 1 à 8, dans laquelle la pathologie neuronale du système nerveux central est une lésion cérébrale due à une ischémie.

11. Composition pour utilisation selon l'une des revendications 1 à 8, dans laquelle la pathologie neuronale du système nerveux central est une lésion cérébrale due à une insuffisance respiratoire.

12. Composition pour utilisation selon l'une des revendications 1 à 8, dans laquelle l'hypoglycémie est due à un diabète.

13. Composition pour utilisation selon l'une des revendications 1 à 8, dans laquelle l'hyperglycémie est due à un diabète.

14. Composition pour utilisation selon l'une des revendications 1 à 8, sous forme d'une solution aqueuse et présentant une concentration de composé de formule (I) comprise entre 1 pmol.L⁻¹ et 1 mmol.L⁻¹, préférentiellement entre 10 pmol.L⁻¹ et 0,1 mmol.L⁻¹, plus préférentiellement entre 0,1 nmol.L⁻¹ et 1 µmol.L⁻¹. 1

## Patentansprüche

1. Zusammensetzung umfassend mindestens eine Verbindung der Formel (I): 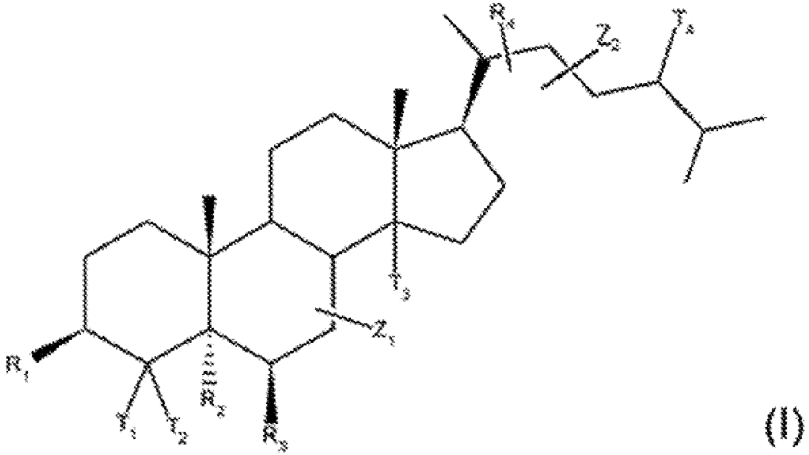 wobei
R₁ =OH;
R₂ =OH;
R₃ = -NR₅R₆,
R₅ H ist, und
R₆ ausgewählt ist aus der Gruppe bestehend aus -(CH₂)₃NH(CH₂)₄NHR₇; - (CH₂)₄NH(CH₂)₃NHR₇; -(CH₂)₃NH(CH₂)₄NH(CH₂)₃NHR₇; -(CH₂)₃NHR₇; -(CH₂)₄NHR₇ mit R₇ = H oder COCH₃; -(CH₂)₂-Imidazol-4-yl; und -(CH₂)₂-Indol-3-yl; und
R₄ = H an der Position 20, 22, 24, 25, 26 oder 27, die so positioniert ist, dass ein asymmetrisches Zentrum mit R- oder S-Konfiguration gebildet ist;
Z₁ und Z₂ jeweils die Anzahl der Doppelbindungen zwischen den Kohlenstoffatomen C7 und C8 beziehungsweise C22 und C23 darstellen; Z₁ ist entweder 0 oder 1; Z₂ = 0; T₁=T_{z}=T₃=T₄=H, wobei T₄ so positioniert ist, dass ein asymmetrisches Zentrum mit R- oder S-Konfiguration an der Position 24 erhalten ist;
und/oder mindestens ein pharmazeutisch annehmbares Salz mindestens einer Verbindung der Formel (I),
zur Verwendung bei der Behandlung einer neuronalen Erkrankung eines Subjekts im Zusammenhang mit Hypoxie, Hypoglykämie und/oder Hyperglykämie, die Zellen des zentralen Nervensystems betreffen.

2. Zusammensetzung zur Verwendung nach Anspruch 1, wobei die Verbindung der Formel (I) durch Z₁ = 0 definiert ist.

3. Zusammensetzung zur Verwendung nach Anspruch 2, wobei die Verbindung der Formel (I) durch R₆ = -(CH₂)₄NH(CH₂)₃NHR₇ mit R₇ = COCH₃ definiert ist.

4. Zusammensetzung zur Verwendung nach Anspruch 2, wobei die Verbindung der Formel (I) durch R₆ = -(CH₂)₂-Imidazol-4-yl definiert ist.

5. Zusammensetzung zur Verwendung nach Anspruch 2, wobei die Verbindung der Formel (I) durch R₆ = -(CH₂)₃NH(CH₂)₄NHR₇, -(CH₂)₄NH(CH₂)₃NHR₇, - (CH₂)₃NH(CH₂)₄NH(CH₂)₃NHR₇; oder -(CH₂)₄NHR₇; et R₇ = H definiert ist.

6. Zusammensetzung zur Verwendung nach Anspruch 1, wobei das Verbindung der Formel (I) durch Z₁ = 1 definiert ist.

7. Zusammensetzung zur Verwendung nach Anspruch 6, wobei die Verbindung der Formel (I) durch R₆ = -(CH₂)₃NH(CH₂)₄NHR₇; -(CH₂)₄NH(CH₂)₃NHR₇; oder - (CH₂)₃NH(CH₂)₄NH(CH₂)₃NHR₇; et R₇ = H definiert ist.

8. Zusammensetzung umfassend mindestens eine Verbindung der Formel (I): 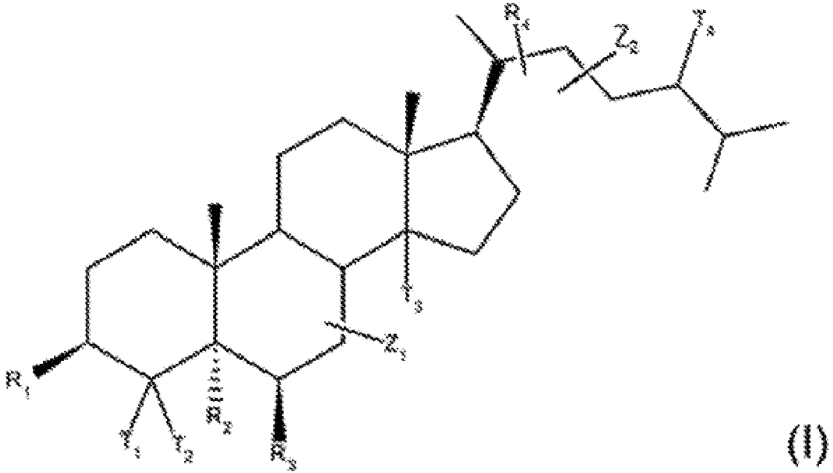 wobei
R₁ = F, OCₙH₂ₙ₊₁, R-COO, R-OCOO, RHN-COO oder OPO(OR)₂ mit R = H oder CₙH₂ₙ₊₁,mit n ≤ 16;
R₂ =OH
R₃ = -NR₅R₆,
R₄ = H oder OH an der Position 20, 22, 24, 25, 26 oder 27, die so positioniert ist, dass ein asymmetrisches Zentrum mit R- oder S-Konfiguration gebildet ist;
R₅= H;
R₆ ausgewählt ist aus der Gruppe bestehend aus -(CH₂)₄NH(CH₂)₃NHR₇, - (CH₂)₃NH(CH₂)₄NHR₇, -(CH₂)₃NH(CH₂)₄NH(CH₂)₃NHR₇; -(CH₂)₄NHR₇; mit R₇ = H oder COCH₃; -(CH₂)₂-Imidazol-4-yl; und -(CH₂)₂-Indol-3-yl;
Z₁ und Z₂ jeweils die Anzahl der Doppelbindungen zwischen den Kohlenstoffatomen C7 und C8 beziehungsweise C22 und C23 darstellen;
Z₁ = 0 oder Z₁ = 1;
Z₂ = 0;
T₁, T₂, T₃ und T₄=H, wobei T₄ so positioniert ist, dass ein asymmetrisches Zentrum mit R- oder S-Konfiguration an der Position 24 erhalten ist;
und/oder mindestens ein pharmazeutisch annehmbares Salz mindestens einer Verbindung der Formel (I),
zur Verwendung bei der Behandlung einer neuronalen Erkrankung eines Subjekts im Zusammenhang mit Hypoxie, Hypoglykämie und/oder Hyperglykämie, die Zellen des zentralen Nervensystems betreffen.

9. Zusammensetzung zur Verwendung nach einem der Ansprüche 1 bis 8, wobei die neuronale Erkrankung des zentralen Nervensystems aus der Gruppe ausgewählt ist, die aus Hirntrauma und Schlaganfall besteht.

10. Zusammensetzung zur Verwendung nach einem der Ansprüche 1 bis 8, wobei die neuronale Erkrankung des zentralen Nervensystems eine durch Ischämie verursachte Hirnverletzung ist.

11. Zusammensetzung zur Verwendung nach einem der Ansprüche 1 bis 8, wobei die neuronale Erkrankung des zentralen Nervensystems eine Hirnverletzung aufgrund von Atemversagen ist.

12. Zusammensetzung zur Verwendung nach einem der Ansprüche 1 bis 8, wobei die Hypoglykämie durch Diabetes verursacht ist.

13. Zusammensetzung zur Verwendung nach einem der Ansprüche 1 bis 8, wobei die Hyperglykämie durch Diabetes verursacht ist.

14. Zusammensetzung zur Verwendung nach einem der Ansprüche 1 bis 8 in Form einer wässrigen Lösung aufweisend eine Konzentration der Verbindung der Formel (I) zwischen 1 pmol ^{∗} L⁻¹ und 1 mmol ^{∗} L⁻¹, bevorzugt zwischen 10 pmol ^{∗} L⁻¹ und 0,1 mmol ^{∗} L⁻¹, weiter bevorzugt zwischen 0,1 nmol ^{∗} L⁻¹ und 1 µmol ^{∗} L⁻¹.

## Claims

1. A composition comprising at least one compound of formula (I): 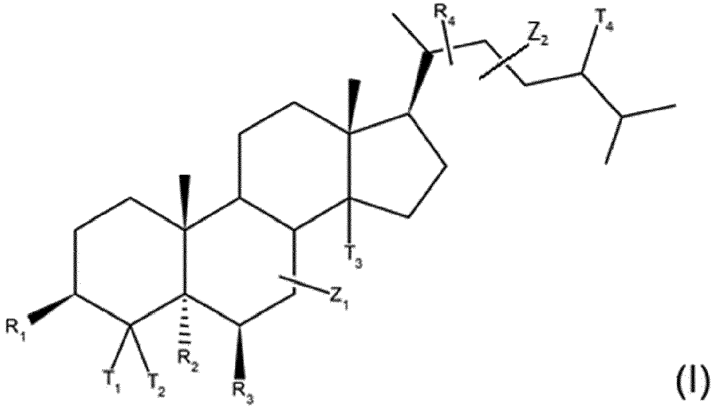 formula wherein
R₁ = OH,
R₂ = OH;
R₃ = -NR₅R₆,
R₅ being H, and
R₆ being taken from the group formed by -(CH₂)₃NH(CH₂)₄NHR₇; - (CH₂)₄NH(CH₂)₃NHR₇; -(CH₂)₃NH(CH₂)₄NH(CH₂)₃NHR₇; -(CH₂)₃NHR₇; -(CH₂)₄NHR₇ with R₇ = H or COCH₃; -(CH₂)₂-imidazol-4-yl and -(CH₂)₂-indol-3-yl; and
R₄ = H in position 20, 22, 24, 25, 26 or 27, positioned so as to create an asymmetric center of configuration R or S;
Z₁ and Z₂ each represent the number of double bonds between the carbon atoms C7 and C8 and C22 and C23 respectively; Z₁ is either 0 or 1; Z₂ =0; T₁ = T₂ = T₃ = T₄ = H; T₄ being positioned so as to obtain an asymmetric center of configuration R or S in position 24;
and/or at least one pharmaceutically acceptable salt of at least one compound of formula (I),
for use thereof in the treatment of a neuronal pathology of a subject, said neuronal pathology being related to hypoxia, to hypoglycemia and/or to hyperglycemia affecting cells of the central nervous system.

2. The composition for use as claimed in claim 1, wherein the compound of formula (I) is defined by Z₁ = 0.

3. The composition for use as claimed in claim 2, wherein the compound of formula (I) is defined by R₆ = -(CH₂)₄NH(CH₂)₃NHR₇ with R₇ = COCH₃.

4. The composition for use as claimed in claim 2, wherein the compound of formula (I) is defined by R₆ = -(CH₂)₂-imidazol-4-yl.

5. The composition for use as claimed in claim 2, wherein the compound of formula (I) is defined by R₆ = -(CH₂)₃NH(CH₂)₄NHR₇, -(CH₂)₄NH(CH₂)₃NHR₇, -(CH₂)₃NH(CH₂)₄NH(CH₂)₃NHR₇; or-(CH₂)₄NHR₇; and R₇ = H.

6. The composition for use as claimed in claim 1, wherein the compound of formula (I) is defined by Z₁ = 1.

7. The composition for use as claimed in claim 6, wherein the compound of formula (I) is defined by R₆ = -(CH₂)₃NH(CH₂)₄NHR₇; -(CH₂)₄NH(CH₂)₃NHR₇; or -(CH₂)₃NH(CH₂)₄NH(CH₂)₃NHR₇; and R₇ = H.

8. A composition comprising at least one compound of formula (I): 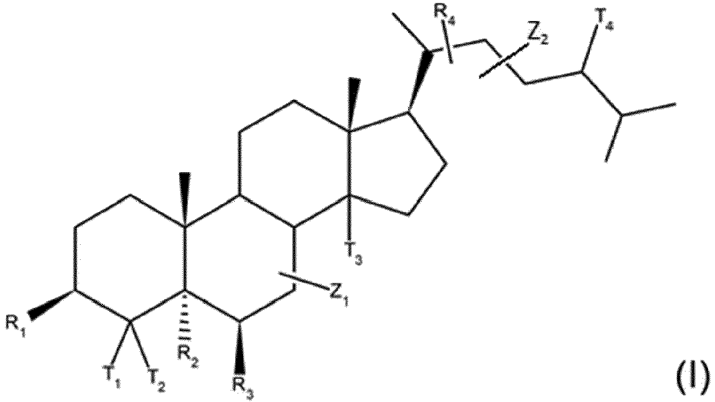 formula wherein
R₁ = F, OCₙH₂ₙ₊₁, R-COO, R-OCOO, RHN-COO or OPO(OR)₂ with R = H or CₙH₂ₙ₊₁, with n ≤ 16;
R₂ = OH;
R₃ = -NR₅R₆,
R₄ = H in position 20, 22, 24, 25, 26 or 27, positioned so as to create an asymmetric center of configuration R or S;
R₅ = H;
R₆ being taken from the group formed by -(CH₂)₃NH(CH₂)₄NHR₇; - (CH₂)₄NH(CH₂)₃NHR₇; -(CH₂)₃NH(CH₂)₄NH(CH₂)₃NHR₇; -(CH₂)₄NHR₇ with R₇ = H or COCH₃; -(CH₂)₂-imidazol-4-yl and -(CH₂)₂-indol-3-yl;
R₄ = H in position 20, 22, 24, 25, 26 or 27, positioned so as to create an asymmetric center of configuration R or S;
Z₁ and Z₂ each represent the number of double bonds between the carbon atoms C7 and C8 and C22 and C23 respectively;
Z₁ is either 0 or 1; Z₂ =0;
T₁ = T₂ = T₃ = T₄ = H; T₄ being positioned so as to obtain an asymmetric center of configuration R or S in position 24;
and/or at least one pharmaceutically acceptable salt of at least one compound of formula (I),
for use thereof in the treatment of a neuronal pathology of a subject, said neuronal pathology being related to hypoxia, to hypoglycemia and/or to hyperglycemia affecting cells of the central nervous system.

9. The composition for use as claimed in one of claims 1 to 8, wherein the neuronal pathology of the central nervous system is chosen from the group consisting of cerebral traumas and strokes.

10. The composition for use as claimed in one of claims 1 to 8, wherein the neuronal pathology of the central nervous system is a cerebral lesion due to ischemia.

11. The composition for use as claimed in one of claims 1 to 8, wherein the neuronal pathology of the central nervous system is a cerebral lesion due to respiratory insufficiency.

12. The composition for use as claimed in one of claims 1 to 8, wherein the hypoglycemia is due to diabetes.

13. The composition for use as claimed in one of claims 1 to 8, wherein the hyperglycemia is due to diabetes.

14. The composition for use as claimed in one of claims 1 to 8, in the form of an aqueous solution and having a concentration of compound of formula (I) of between 1 pmol.L⁻¹ and 1 mmol.L⁻¹, preferentially between 10 pmol.L⁻¹ and 0.1 mmol.L⁻¹, more preferentially between 0.1 nmol.L⁻¹ and 1 µmol.L⁻¹.
